# EUROPEAN PATENT APPLICATION

(11) **EP 3 778 916 A1**
(43) Date of publication of application: **17.02.2021**
(21) Application number: 19777053.0
(22) Date of filing: 01.04.2019
(51) Int. Cl.: C12P 21/06, C07K 14/78, C07K 14/82, C07K 16/30, C12P 21/08, C12Q 1/34, G01N 33/53

(54) **ANTIGEN TREATMENT METHOD**

(30) Priority: 30.03.2018 JP 2018069054; 29.03.2019 WO PCT/JP2019/014295
(71) Applicant: Sekisui Medical Co., Ltd., Tokyo 103-0027 (JP)
(72) Inventor: MIURA Tomohiro, Tokyo 103-0027 (JP); SHIMIZU Tomo, Tokyo 103-0027 (JP); MIYAZAKI Osamu, Tokyo 103-0027 (JP)
(74) Representative: Hoffmann Eitle
(86) International application number: PCT/JP2019/014554
(87) International publication number: WO 2019/189940

(57) **Abstract**

What is required is a method of producing, without treating a glycoprotein in a biological sample with a denaturing agent in advance, a selectively binding material, which specifically reacts with a glycan of a glycoprotein, a selectively binding material such as antibody thus produced by the method, and a method of specifically detecting a glycoprotein by using the selectively binding material.

In order to solve the problems, the present invention produces a selectively binding material specifically reactive with a glycan of a glycoprotein by treating the glycoprotein with a glycan-specific glycan-cleaving enzyme.

## Description

### Technical Field

The present invention relates to a method of treating an antigen for use in producing a selectively binding material such as an antibody, a method of producing a selectively binding material such as an antibody by using the antigen thus treated, a selectively binding material such as an antibody, thus produced by the method, a method of detecting a glycoprotein by using the selectively binding material such as an antibody, and a kit for use in these methods.

### Background Art

Glycoproteins have various glycans varied in structure, and therefore, if it becomes possible to utilize an antibody capable of recognizing such glycans individually, it would make it possible to obtain various information on states of living bodies and the like.

On the other hand, it has been known that obtaining an antibody capable of specifically binding with a glycan of a glycoprotein is difficult, and it is considered that the presence of a similar glycan in a host animal producing the antibody is one of reasons for the difficulty. Various efforts have been made to overcome the difficulty. For example, Patent Document 1 discloses a method of producing an antibody by using an antigen having both of a fucose portion and a peptide portion of a glycopeptide, the antibody being capable of specifically binding with the fucose portion and the peptide portion as epitopes thereof.

However, this method is not so practical because, in order to detect a glycoprotein in a sample by using an antibody produced by this method, it is necessary to denature the glycoprotein by treating the biological sample in advance with a denaturing agent such as SDS.

Moreover, Patent Document 2 discloses a method of increasing a detection level of detecting a reactant in a reaction between a glycoprotein and a saccharide-binding compound, the method increasing the detection level by treating the glycoprotein with a proteinase.

However, this method is not capable of specifically detecting a glycoprotein having a certain glycan, because an activity of the proteinase is not specific to a glycan structure.

Therefore, there has been a demand for a widely-applicable method for preparing an antibody specifically reactive with a glycan of glycoprotein without treating a biological sample with a denaturing agent in advance.

### Citation List

### Patent Literatures

Patent Document 1: JP 2017-214348 A
Patent Document 2: WO 2017/131022

### Summary of Invention

### Technical Problem

The present invention provides a method of producing, without treating a biological sample with a denaturing agent in advance, a selectively binding material such as antibody, which specifically reacts with a glycan of a glycoprotein, a selectively binding material such as antibody thus produced by the method, and a method of specifically detecting a glycoprotein by using the selectively binding material such as antibody.

### Solution to Problem

In order to solve the problems, the present inventors diligently studied and found that an antibody specifically reactive with a glycan of a glycoprotein can be obtained by treating the glycoprotein with a glycan-specific glycan-cleaving enzyme under a moderate condition, thereby accomplishing the present invention.

Without wishing to be bound by any theory, it is considered that the reason for this phenomenon is that a glycan produces a glycan structure very rare in an animal living body such as a fucosylated glycopeptide after the glycan is treated with the glycan-specific glycan-cleaving enzyme, and therefore antibody production in the animal producing the antibody will not be adversely affected.

Therefore, the present invention provides the followings according to exemplary aspects thereof.
[Item 1] A method of treating a glycoprotein when the glycoprotein is used as an antigen,
   the method including:
   a step of cleaving a glycan with a glycan-specific glycan-cleaving enzyme.
[Item 2] The method of treating a glycoprotein according to Item 1,
   in which the step of cleaving the glycan
   causes an epitope present in the glycan to be recognizable with a selectively binding material.
[Item 3] The method of treating a glycoprotein according to Item 1 or 2, in which the glycan-specific glycan-cleaving enzyme is one selected from the group consisting of Endo-A, Endo-M, Endo-H, Endo-D, Endo-F1, Endo-F2, and Endo-F3.
[Item 4] The method of treating an antigen according to any one of Items 1 to 3, in which the glycoprotein is AFP.
[Item 5] A method of producing a selectively binding material, in which the method of treating a glycoprotein according to any one of Items 1 to 4 is employed.
[Item 6] A method of detecting a glycoprotein in a biological sample, in which the method of treating a glycoprotein according to any one of Items 1 to 4 is employed.
[Item 7] A glycoprotein treated by the method of treating a glycoprotein according to any one of Items 1 to 4.
[Item 8] A selectively binding material being bindable with a glycoprotein treated by the method of treating an antigen according to any one of Items 1 to 4 and not bindable with the same glycoprotein not treated as such.
[Item 9] The selectively binding material according to Item 8, being an antibody or an antigen-binding fragment of an antibody.
[Item 10] A method of producing the selectively binding material according to Item 8 or 9, the method including: employing, as an antigen, a glycoprotein treated with the method of treating an antigen according to any one of Items 1 to 4.
[Item 11] A method of producing the selectively binding material according to Item 8 or 9, the method including: employing, as an antigen, a glycoprotein obtained by chemical synthesis or gene recombination technique.
[Item 12] A method of detecting a glycoprotein,
   the method including:
   a step of treating, with a glycan-cleaving enzyme, a sample that potentially contains the glycoprotein;
   a step of applying the selectively binding material according to Item 8 to the glycoprotein treated with the glycan-cleaving enzyme, so as to obtain a reaction product of a reaction between the glycoprotein and the selectively binding material; and
   a step of detecting the reaction product.
[Item 13] The method according to Item 12, in which the step of treating the glycoprotein with the glycan-specific glycan-cleaving enzyme and the step of obtaining the reaction product of the reaction between the glycoprotein and the selectively binding material are carried out at the same time.
[Item 14] A reagent kit for use in the method according to Item 12 or 13, in which a reagent for treating a glycoprotein with a glycan-specific glycan-cleaving enzyme and a reagent for obtaining a reaction product of the glycoprotein and a selectively binding material are included in a single composition.
[Item 15] A method of producing an antibody by using an antigen having a glycan structure not present in vivo or very rare in vivo even if the glycan structure is present in vivo.
[Item 16] The method according to Item 15, in which the antigen is a glycoprotein treated with a glycan-specific glycan-cleaving enzyme.
[Item 17] The method according to Item 15, in which the antigen is one obtained by chemical synthesis or gene recombination technique.

### Advantageous Effects of Invention

The use of the method of treating a glycoprotein according to the present invention makes it possible to produce, merely by a moderate-conditioned treatment without denaturing the glycoprotein in a sample, a selectively binding material such as an antibody for detecting the glycoprotein.

Furthermore, the method of detecting a glycoprotein by using the selectively binding material such as an antibody thus produced by the method according to the present invention can be employed in combination with another detection technique that cannot be used under a denaturing condition, because the method of detecting according to the present invention can be employed without denaturing a biological sample.

### Brief Description of Drawings

Fig. 1 is a view schematically illustrating a method of producing an antibody by treating a glycoprotein with a glycan-specific glycan-cleaving.
Fig. 2 is a view illustrating cleavage of a glycan by treating a glycoprotein with a glycan-specific glycan-cleaving enzyme.
Fig. 3 is a view schematically illustrating a glycopeptide.
Fig. 4 is a view illustrating results of treatment of AFP with Endo-F2 or Endo-F3. Fig. 4 demonstrates that both of these enzymes are capably of digesting a glycan.
Fig. 5 is a view illustrating a result of an antigen-immobilized ELISA method with an antiserum of an immunized animal in case where an immunogen in which a disaccharide peptide was linked with a protein was used. Fig. 5 demonstrates that a greater concentration of an antibody specific to the disaccharide peptide was contained in the antiserum of the immunized animal than in a serum of an unimmunized animal.
Fig. 6 is a view illustrating a result of an antigen-immobilized ELISA method with a culture supernatant of an anti-AFP antibody-producing hybridoma. Fig. 6 demonstrates that a plurality of antibody-producing strains reactive with the disaccharide peptide but not with a monosaccharide peptide was confirmed in the animal inoculated with the disaccharide peptide.
Fig. 7 is a view illustrating a result of an antigen-immobilized ELISA method with an antiserum of an immunized animal in case where an immunogen prepared by purifying AFP from a culture supernatant of HepG2, treating the AFP with Endo-F3 for enzyme removal was also used. Fig. 7 demonstrates that a greater concentration of an antibody specific to the disaccharide peptide was contained in the antiserum of the immunized animal than in a serum of an unimmunized animal.
Fig. 8 is a view illustrating a result of an antigen-immobilized ELISA method with an anti-AFP antibody-producing hybridoma. Fig. 8 demonstrates that a plurality of antibody-producing strains reactive with the disaccharide peptide but not with a monosaccharide peptide was confirmed in the animal inoculated with the disaccharide peptide.
Fig. 9 is a view illustrating a result of a specificity analysis of a monoclonal antibody. Fig. 9 demonstrates that S20205R antibody or S20206R antibody recognizes only glycolytic AFP produced by an enzymic treatment.
Fig. 10 illustrates a result of treatment of AFP with Endo-F3 or PNGaseF. Fig. 10 demonstrates that AFP was digested with either of these enzymes.
Fig. 11 is a view illustrating a result of a specificity analysis of a monoclonal antibody. Fig. 11 demonstrates that the S20205R antibody and S20206R antibody contain, as a part of a recognition site, a glycan produced by treatment with Endo-F3.
Fig. 12a is a view illustrating a result of sandwich ELISA with an antibody of the present invention and a conventional antibody. Fig. 12a demonstrates that the use of S20205R antibody increased absorbance dependently of the concentration of the enzyme-treated AFP.
Fig. 12b is a view illustrating a result of sandwich ELISA with an antibody of the present invention and a conventional antibody. Fig. 12b demonstrates that the use of S20206R antibody as a labelled detection antibody increased absorbance dependently of the concentration of the enzyme-treated AFP.
Fig. 13a is a view illustrating a result of a reactivity analysis with respect to enzyme treatment products. Fig. 13a demonstrates that the use of a conventional anti-AFP antibody as a labelled detection antibody in ELISA analysis increased absorbance in an AFP concentration dependent manner regardless of whether or not the enzyme treatment was carried out.
Fig. 13b is a view illustrating a result of a reactivity analysis with respect to enzyme treatment products. Fig. 13b demonstrates that the use of S20206R antibody increases absorbance dependently of the concentration of only the AFP treated with Endo-F2 or Endo-F3.
Fig. 14 is a view illustrating a result of an enzyme treatment in blood serum. Fig. 14 demonstrates that Endo-F3 also works on the AFP even in blood serum, thereby making it possible to detect AFP treated with the enzyme treatment with the antibody of the present invention.
Fig. 15 is a view illustrating a result of a study on pH condition in the enzyme treatment. Fig. 15 demonstrates that the enzyme for use in the present invention can be used without a particular pH requirement.
Fig. 16 is a view illustrating a result of an on-plate enzyme treatment. Fig. 16 demonstrates that antigen-antibody reaction and the antigen-enzyme reaction can be performed at the same time.

### Description of Embodiments

In the followings, embodiments of the present invention will be described. It should be understood that the following explanations are merely for illustrative purposes and the scope of the present invention is not limited by these explanations and can be put into practice in a manner modified as appropriate within the gist of the present invention.

### (Definitions)

Unless otherwise specified, all technical and scientific terms used in this specification have the meanings as understood by the person skilled in the art to which the present invention pertains.

In this specification, in case where a plurality of numerical ranges is presented, it means that ranges between arbitrary combinations of a lower limit of one of the plurality of numerical ranges and an upper limit of another one of the plurality of numerical ranges are also encompassed.

In this specification, what is meant by the term "glycoprotein" is a protein containing one or more carbohydrate groups attached with a polypeptide chain via covalent bonding. For example, the glycoprotein contains carbohydrates in the forms of a lot of branched relatively-short oligoglycans with non-uniform compositions by 1wt% to 60wt%.

Most of natural peptides and proteins contain a carbohydrate portion that bonds with a peptide or a protein via bonding specific to a selected number of amino acids along a length of a main peptide or protein chain. Specificity of glycosylation pattern on a peptide or protein would possibly affect the function of the peptide or protein. For example, a structure of an N-bonding glycan on a peptide or protein would possibly affect various properties of the peptide or protein such as protease sensitivity, intracellular transport, secretion, tissue targeting, biological half-life, and antigenicity of a peptide or protein in a cell or living organism.

A change in one or more of these properties would possibly affect effectiveness of the peptide or protein in the natural environment where the change occurs.

Moreover, for example, it is known that glycoprotein AFP having a peptide including 590 amino acids is present in the forms of AFP-L1 and AFP-L3 having different glycan structures in vivo. It is known that, among these forms of AFP, only AFP-L3 is present specifically in case of liver cancer.

Therefore, by using an antibody for specifically detecting the glycan of AFP-L3, it is possible to detect whether or not liver cancer is present in vivo.

In this specification, what is meant by a phrase "not present in vivo or very rare even if the molecule is present in vivo" in regard to a particular in-vivo molecule is that a molecular weight or weight of the in-vivo molecule present in a living body in an ordinary condition is not detectable with an ordinary detecting means, or is low compared with similar in-vivo molecules. What is meant by the living body in the ordinary condition is a living body in a resting state without a particular disease, disorder, or the like, and what is meant by the word "low" is, for example, that the molecular weight or weight of the in-vivo molecule is 1/100, 1/1000, or 1/10000 or less of that of the similar in-vivo molecules. For example, in case where a glycoprotein is "not present in vivo or very rare in vivo even if the molecule is present in vivo," what is meant by this phase is that an amount of this glycoprotein present in vivo is low compared with a glycoprotein having the same protein in its composition but having a different glycan structure. For example, hexasaccharide, tetrasaccharide, or disaccharide glycoproteins (for examples, fucosylated disaccharide glycopeptide) are very rare in vivo.

In this specification, the word "detecting" or "detection" is used with wide meaning encompassing qualitative measuring or measurement and quantitative measuring or measurement of target molecules. The detection encompasses direct and indirect detection, and encompasses any means for detecting.

According to one aspect, an antibody of the present invention is used for a method of detecting the presence of a glycoprotein in a biological sample. According to one embodiment, the method includes a step of contacting an antibody of the present invention with a biological sample under a condition where bonding between the antibody of the present invention and a glycoprotein is allowed, and detecting whether or not a complex of the antibody and the glycoprotein is formed. Such a method may be in-vitro or in-vivo. According to one embodiment, a biomarker indicating a state of a patient is detected by using the antibody of the present invention.

In this specification, expressions such as a selectively binding material such as an antibody "reacts," is reactive," "has reactivity," or "bonds" with a compound, or a selectively binding material such as an antibody "recognizes" a compound encompass meanings usually used in the field to which the present invention pertains, and these expressions are synonymously usable. Confirmation as to whether or not a selectively binding material such as an antibody "reacts" with a compound can be carried out by techniques well-known to a person skilled in the art such as an antigen-immobilized ELISA method, a competitive ELISA method, and a sandwich ELISA method, while the confirmation can be performed by a method using the principal of the surface plasmon resonance (SPR method) or the like method. The SPR method can be carried out by using a device, a sensor, and reagents, which are commercially available under the name of Biacore (registered trademark).

In this specification, what is meant by a phase a selectively binding material such as an antibody according to the present invention "does not react" with a compound is that the selectively binding material such as an antibody according to the present invention does not substantially react with a compound. What is meant by the expression "not substantially reacting" is that, for example, in the antigen-immobilized ELISA method, the addition of the compound does not substantially affect the bonding between the selectively binding material such as an antibody and the immobilized antigen. The "not substantially reacting" can be confirmed also by a method or means well-known for a person skilled in the art other than the antigen-immobilized ELISA method.

In this specification, what is meant by the phase "a selectively binding material such as an antibody "specifically reacts" or the word "specificity" of the selectively binding material such as an antibody is an ability of reacting with an epitope presented on the antigen in such a manner that the selectively binding material such as an antibody can be detected but reactivity with the other antigens is relatively limitedly detectable or substantially not detectable. For example, in case where a selectively binding material such as an antibody "specifically reacts" with a certain antigen, the selectively binding material such as an antibody reacts with the antigen but not with the other antigens. In preferable aspects, in case where a selectively binding material such as an antibody "specifically reacts" with a certain antigen, for example, interaction between the antigen immobilized in the antigen-immobilized ELISA method and the selectively binding material such as the antibody is hindered by the antigen being free but not by the other antigens being free.

In this specification, what is meant by a "reaction product" between a selectively binding material and an antigen is a material that is produced as a result of specifically reacting the selectively binding material with the antigen and is detectable by an ordinary detection method.

In this specification, what is meant by a "selectively binding material" or an "antigen-binding material" is a natural or non-natural material that is capable of specifically binding with a certain material and has the same usage as antibody. For instance, examples of the selectively binding material include aptamers such as peptide aptamers, DNA aptamers, and RNA aptamers, lectins, receptors, modified aptamers, lectins, and receptors, and combinations thereof.

In this specification, what is meant by the word "antigen" a molecule or part of a molecule, which is capable of receiving bonding with a selectively binding material such as an antibody. The antigen can have one or more epitopes, each of which may interact with a different selectively binding material, for example, antibody. The "antigen" can be used not only for the production of selectively binding materials such as antibodies, but also as reference standards when measuring glycoproteins.

In this specification, what is meant by the word "epitope" is one region of an antigen, to which a selectively binding material targeting the antigen binds.

In this specification, what is meant by the "antibody" is an immunoglobulin molecule including two heavy chains (H) and two light chains (L) connected with each other via four polypeptide chains and disulfide bonding. Each of the heavy chains includes a heavy chain variable region ("HCVR" or "VH") and a heavy chain constant region (including CH₁, CH₂, and CH₃ domains) . Each of the light chains includes a light chain variable region ("LCVR" or "VL") and a light chain constant region (CL). The VH and VL regions may further be divided into hypervariable regions named as complementary-determining regions (CDR), which are scattered within regions named as frameworks (FR) where many of them can be stored. Each VH and VL includes three CDRs and four FRs, and is arranged from the amine terminal to the carboxy terminal in the following order: FR1, CDR1, FR2, CDR2, FR3, CDR3, and FR4. The variable regions of the heavy chains and the light chains include a binding domain. The term "antibody" also includes all genetically recombinant antibodies of the antibody, for example, antibodies expressed in prokaryote and antibodies not glycosylated.

Moreover, as described in Padlan (1995 FASEB J. 9:133-139), Vajdos et al. 2002 J Mol Biol 320:415-428, it is known that only some of the CDR residues contacts with the antigen in reality. CDR residues not in contact with the antigen can be identified by molecular modeling or by experience from CDR regions of Kabat being present outside CDR of Chothia. In case where CDR or one or more of residues thereof are removed, the CDR or one or more of residues thereof are substituted in general with another human antibody sequence or an amino acid that occupies a corresponding site in consensus of such a sequence. Where to substitute in the CDR and the amino acid can be also selected by experience. The experiential substitution may be conservative substitution or nonconservative substitution.

In this specification, what is meant by an "antigen-binding fragment" of an antibody is one or more fragments of the antibody, which maintain the ability of specifically binding with the antigen. Non-restrictive examples of such binding fragments encompassed by the "antigen-binding fragment" of an antibody include: (i) Fab fragment, which is a monovalent fragment including VL, VH, CL, and CH domains; (ii) F(ab')₂ fragment, which is a divalent fragment including two Fab fragments bonded via a disulfide bridge at a hinge region; (iii) Fd fragment including VH and CH domains; (iv) Fv fragment including VL and VH domains of a single arm of an antibody; (v) dAb fragment including a VH domain (Ward et al., (1989) Nature 341:544-546); (vi) isolated complementary-determining region (CDR); and (vii) combinations of two or more isolated CDRs, which may be linked via a synthetic linker. Moreover, such binding fragments may be produced as a single protein chain, as being known as a single chain Fv (scFv), in which VL and VH regions are formed in pair by using a synthetic linker with the use of gene recombination method (Bird et al. (1988) Science 242: 423-426; and Huston et al. (1988) Proc. Natl. Acad. Sci. USA 85: 5879-5883). Moreover, the "antigen-binding fragment" may be binding domain immunoglobulin fused protein including: (i) a binding domain polypeptide fused with an immunoglobulin hinge region polypeptide; (ii) an immunoglobulin heavy chain CH2 constant region fused with a hinge region; and (iii) an immunoglobulin heavy chain CH3 constant region fused with a CH2 constant region. These antibody fragments may be obtained by a conventional technique well-known to a person skilled in the art.

The antibody usable in the present invention or an antigen-binding fragment thereof may be derived from any animal origin including birds and mammals. Preferably, the antibody or fragment may be derived from human, chimpanzee, rodent animal (such as mouse, rat, guinea pig, or rabbit), chicken, turkey, pig, sheep, goat, camel, cow, horse, donkey, cat, or dog origin. The antibody of the present invention encompasses chimeric molecules in which a constant region of an antibody derived from a certain species is combined with an antigen-binding site derived from another species. Furthermore, the antibody of the present invention encompasses humanized molecules in which an antigen-binding site of an antibody derived from a non-human species (such as mouse origin), a constant region derived from human origin, and a framework region.

The antibody of the present invention may be obtained from a hybridoma expressing the antibody or a host cell expressing the antibody as a result of genetic recombination. As the host cell, a CHO cell, a lymphocyte cell, a bacteria cell such as E. coli, and fungi cell such as yeast.

Moreover, the antibody of the present invention may be produced in a non-human animal or a plant, which has been subjected to gene transfer by using a gene recombination technique. In one aspect, a gene of a hybridoma producing a desired antibody is analyzed and an antibody having an amino acid sequence having the same light chain variable region and heavy chain variable region as the desired antibody is expressed from another host cell by using the gene recombination technique.

As the antibody of the present invention, for example, a monoclonal antibody produced by hybridoma S20205R or S20206R is preferable. The hybridomas are internationally deposited under the Budapest Treaty as below.

Name of the depositary institution: The National Institute of Technology and Evaluation, the NITE Patent Microorganisms Depositary, Address of the depositary institution: Room 122, 2-5-8, Kazusa Kamatari, Kisarazu City, Chiba Pref., (Post code 292-0818), Date of deposition: March 22, 2019
Accession number: NITE BP-02926 (Hybridoma S20205R)
Accession number: NITE BP-02927 (Hybridoma S20206R)

The glycan-specific glycan-cleaving enzyme for use in the present invention is not particularly limited and various well-known enzymes for cleaving a glycan of a glycoprotein present in vivo may be used as the glycan-cleaving enzyme, and combinations of a plurality of enzymes may be used as the glycan-cleaving enzyme. In one aspect, it is preferable that a glycan-specific glycan-cleaving enzyme or a combination of glycan-specific glycan-cleaving enzymes be such that treatment therewith produces a glycan of 2, 3, 4, 5, 6, 7, 8, 9, 10, 11, or 12 in length.

In one aspect, it is preferable that such a glycan-specific glycan-cleaving enzyme be a glycan-cleaving enzyme that selectively recognizes a structure including N,N'-diacetyl chitobiose constituting a basic skeleton of a N-binding-type glycan (GlcNAcβ1, 4 GlcNAc), and that cleaves and releases the glycan in such a way that one residue of N-acetyl glucosamine is left on the protein side (fucose may be attached with the reside). It is especially preferable to employ an enzyme that recognizes core-fucose of a glycan of a glycoprotein and selectively cleaves the glycan in such a way that a glycan having two saccharides including the core-fucose is left on the protein side.

In one aspect, for example, an endoglycosidase may be used as such a glycan-specific glycan-cleaving enzyme. Especially, it is preferable that an endoglycosidase selected from enzymes belonging to endo-β-N-acetylglucosaminidase (EC3.2.1.96) be employed. The selecting of the enzyme can be performed by studying an enzyme reaction condition as much as a person skilled in the art generally performs. By general-purpose analysis method, it is possible to confirm whether or not the desire glycan cleavage has been done by the enzyme reaction. For example, the confirmation can be carried out by analyzing a material produced as a result of the enzyme reaction by mass-spectrometer or the like.

As concrete but non-restrictive examples of such a glycan-specific glycan-cleaving enzyme, an endo-β-N-acetylglucosaminidase such as Endo-A, Endo-M, and Endo-H derived from Streptomyces plicatus, Endo-D derived from Streptococcus pneumoniae, Endo-F1, Endo-F2, and Endo-F3 derived from Flavobacterium meningosepticum may be employed. In one aspect, it is preferable to employ Endo-F1, Endo-F2, or Endo-F3 derived from Flavobacterium meningosepticum, and it is more preferable to employ Endo-F3.

Conditions for treating the glycoprotein in a sample with the glycan-specific glycan-cleaving enzyme may be varied as appropriate for the enzyme to be employed. For example, the treatment may be carried out by mixing the glycoprotein with the glycan-specific glycan-cleaving enzyme at pH in a range of 5 to 8 or 6 to 7 at a temperature in a range of 25 to 40°C, 28 to 39°C, 30 to 37°C, 32 to 36°C, or 33°C to 35°C or at 34°C for a time period in a range of 5 to 60 min, 7 to 50 min, 10 to 40 min, or 15 to 30 min.

A glycoprotein having a glycan structure of the present invention can be used as an antigen for producing a selectively binding material such as an antibody, and also can be used as a standard product in measuring a glycoprotein.

### (Method for producing an antibody specifically reactive with a glycan structure of a glycoprotein of the present invention)

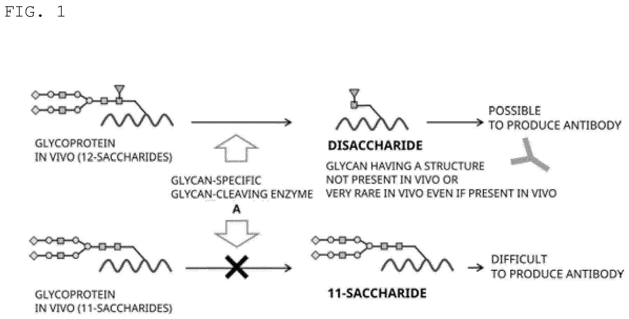

By using, as an antigen, a glycoprotein of the present invention having a glycan structure being thus produced by the aforementioned method and being not present in vivo or very rare in vivo even if the glycan structure is present in vivo, it is possible to produce an antibody specifically reactive with the glycan structure. Fig. 1 schematically illustrates the method of producing the antibody specifically reactive with the glycan of the glycoprotein of the present invention.

More specifically, by treating a glycoprotein with a glycan-specific glycan-cleaving enzyme, a glycan structure not present in vivo or very rare in vivo even if the glycan structure is present in vivo is produced, and the structure is used as an antigen, thereby making it easier to produce an antibody. The antigen is inoculated in a mammal such as a mouse by a known method such as one described in Antibodies, A Laboratory Manual (Cold Spring Harbor Laboratory Press, (1988)). By removing spleen cells or lymph node cells of the animal, and the cells are fused with myeloma cells, thereby producing hybridomas. It is possible to obtain a monoclonal antibody by isolating, from the hybridoma cell groups thus produced, those reactive with the antigen.

In case where the antigen having the glycan structure of the present invention is used as an immunogen, an antigen thus produced by the treatment with the glycan-cleaving enzyme may be used as such or the antigen may be used in such a form that the antigen is bound with a general carrier. Such a single body and a method of binding the antigen and the single body are well-known to a person skilled in the art, for example, the antigen may be bound with a protein such as KLH (Thermo Ltd. 77600) as the carrier via a linker such as Sulfo-SMCC (Thermo Ltd. 22322).

Apart from the antigen thus obtained by treating the glycoprotein with the glycan-specific glycan-cleaving enzyme as described above, the antigen having the glycan structure of the present invention may be a compound having the same structure as the antigen produced by the present invention, the compound being obtained by a well-known method such as chemical synthesis or gene recombination technique. Moreover, the antigen may be an antigen modified in a portion other than epitope. For example, a glycopeptide having the same amino acid and sugar chain sequence as the site that binds to a selective binding substance, which is present in the glycoprotein obtained by a method of treating a glycoprotein with a glycan-specific glycan-cleaving enzyme, can be used as an antigen. These glycopeptides and compounds obtained by chemical synthesis or gene recombination that can be used as antigens can be used not only for the production of selective binding substances such as antibodies, but also as reference standards when measuring glycoproteins.

The preparation of the hybridoma may be performed according to a well-known method in this field, and for example, polyethylene glycol method, a method using Sendai virus, a method using an electric current, or the like may be adopted for the preparation of the hybridoma. The hybridoma thus obtain may be multiplied according to a well-known method, and it is possible to select a desired hybridoma while confirming properties of the antibody produced therefrom. Cloning of the hybridoma may be carried out by a well-known method such as, for example, a limiting dilution method or a soft agar method.

Apart from directly producing from the hybridoma, the antibody thus obtained may be obtained by gene recombination to prepare host cells expressing the antibody, and preparing the antibody from the host cells. As the host cell, a CHO cell, a lymphocyte cell, a bacteria cell such as E. coli, and fungi cell such as yeast.

### (Other methods of producing the selectively binding material)

Apart from producing the antibody, the glycoprotein thus obtained by the method of the present invention for treating a glycoprotein can be used to produce other selectively binding materials which specifically select said glycoprotein, for example, aptamers such as peptide aptamers, DNA aptamers, and RNA aptamers, lectins, receptors, modified aptamers, lectins, and receptors, and combinations thereof. Again, for this purpose, the glycoprotein may be a compound having the same structure produced by a well-known method such as chemical synthesis or gene recombination, apart from the glycoprotein produced by the method treating with the glycan-specific glycan-cleaving enzyme, or may be a glycoprotein obtained by modifying the glycoprotein or the compound.

As a nucleic aptamer such as the DNA aptamers and RNA aptamers, an aptamer that specifically binds with the glycoprotein thus obtained by the method of the present invention for treating a glycoprotein can be obtained by a method well-known to a person skilled in the art such as repeating rounds of Systematic Evolution of Ligands by Exponential Enrichment (SELEX) method (Archemix, Cambridge, MA, USA) (Sampson, 2003), but the preparation of a nucleic aptamer is not limited to this.

As the peptide aptamer, an aptamer that specifically binds with the glycoprotein thus obtained by the method of the present invention for treating a glycoprotein can be obtained by a well-known method such as the method disclosed in Lu et al., Chem Rev 2009: 109(5): 1948-1998 or the like, but the preparation of a nucleic aptamer is not limited to this.

In case where a lectin is used as the selectively binding material, the lectin used may be obtained from arbitrary organism such as plants, animals, yeasts, bacteria, and protozoa. Lectin may be isolated from a naturally-present origin as these, or may be prepared by gene recombination expression and purification according to a method well-known to a person skilled in the art. Moreover, a purified lectin commercially available may be employed. As a selectively binding material specifically that binds with a glycan having fucose, for example, a fucose-specific lectin (see Mansour MH et al. (2005) Immunobiology. 210: 335-48 and the like) may be used. In order to obtain a lectin that specifically binds with the glycoprotein obtained by the method of the present invention for treating a glycoprotein, such a lectin specifically binding the glycoprotein of the present invention may be selected by screening from lectins that have been already obtained, or from modified lectins that specifically bind with the glycoprotein of the present invention by using gene recombination technique. For the same purposes as the lectin, a protein having a lectin-like domain (see Drickamer K (1999) Curr. Opin. Struct. Biol. 9:585-90 and the like) may be isolated and employed.

In case where the receptor is used as a selectively binding material, a receptor that specifically binds with the glycoprotein of the present invention may be selected by screening from known glycoprotein-binding receptors, or from modified receptors that bind with the glycoprotein of the present invention by using gene recombination technique. For the same purpose of the receptor, a modified receptor in which part of the receptor is bound with another protein such as an Fc region of an antibody may be isolated and employed.

### (Applications of the selectively binding material of the present invention)

The selectively binding material such as an antibody thus obtained by the method of the present invention can be used to detect, in a biological sample, a glycoprotein functioning as a biomarker or the like.

### (Method of specifically detecting a glycoprotein in a sample)

In performing a method of the present invention for specifically detecting a glycoprotein in a sample, the sample that potentially contained the glycoprotein may be treated with a glycan-specific glycan-cleaving enzyme. The glycan-specific glycan-cleaving enzyme may be identical with or other than the glycan-specific glycan-cleaving enzyme used in preparing the selectively binding material such as the antibody. In one aspect, it is preferable that the same enzyme or the same combinations of enzymes be employed.

In using the glycan-cleaving enzyme, the enzyme treatment may be performed by adding the glycan-specific glycan-cleaving enzyme into the sample that potentially contained the glycoprotein, or the enzyme treatment with the glycan-cleaving enzyme may be performed after the glycoprotein is purified from the sample to some extent. Moreover, a step of obtaining a reaction product from the reaction between the selectively binding material and the glycoprotein may be carried out after the step of treating the glycoprotein with the glycan-cleaving enzyme for the enzyme treatment, or the step of obtaining a reaction product from the reaction between the selectively binding material and the glycoprotein and the step of treating the glycoprotein with the glycan-cleaving enzyme for the enzyme treatment may be performed at the same time. In one aspect, it is preferable that the step of obtaining a reaction product from the reaction between the selectively binding material and the glycoprotein and the step of treating the glycoprotein with the glycan-cleaving enzyme for the enzyme treatment be performed at the same time. In case where the step of obtaining a reaction product from the reaction between the selectively binding material and the glycoprotein and the step of treating the glycoprotein with the glycan-cleaving enzyme for the enzyme treatment are performed at the same time, it is preferable to select, as the selectively binding material, a material that will not be affected with the glycan-cleaving enzyme.

As a method of detecting a reaction product obtained as a result of the specific reaction between the selectively binding material and the antigen, a well-known method may be used. For example, Enzyme-Linked Immunosolvent Assay (ELISA), Radiommunoassay (RIA), Immunofluorescent measurement, immunoprecipitation, equilibrium dialysis method, immune diffusion method, Immunoaggregation measurement method, immuno - nephelometry, particle immunoassay method and the other techniques, but the method of detecting is not limited to these (see, for example, Harlow and Lane, Antibodies: A Laboratory Manual, Cold Spring Harbor Laboratory, 1988; Weir, D. M., Handbook of Experimental Immunology, 1986, Blackwell Scientific, Boston). In one aspect, the selectively binding material such as an antibody of the present invention may be used as an immobilized (solid phase) material immobilized on an insoluble carrier, or a labelled material (for example, detection antibody) labelled with a labelling material. Such an immobilized selectively binding material or a labelled selectively binding material are also included within the scope of the present invention. For example, an immobilized selectively binding material may be produced by physically adsorbing or chemically bonding the selectively binding material of the present invention onto the insoluble carrier (where the chemical bonding may involve an appropriate spacer between the selectively binding material and the insoluble carrier). As the insoluble carrier, an insoluble carrier made from a polymer material such as polystyrene resin, an inorganic material such as glass, or polysaccharide material such as cellulose and agarose. The insoluble carrier is not limited in terms of its shape and may have an arbitrary shape such as a platelike shape (for example, microplate or membrane), a beads or fine particle-like shape (for example, latex particles or magnetic particles), or a tube-like shape (for example, test tube).

Examples of the labelling material for producing the labelled selectively binding material include enzymes, fluorescent materials, chemically light emitting materials, biotin, avidin, radioactive isotope, gold collide particles, color latex, and the like. As a method of binding the labelling material and the selectively binding material, a glutaraldehyde method, a maleimide method, a pyridyl disulfide method, or a periodic acid method, or the like available for a person skilled in the art may be adopted. The immobilized selectively binding material and the labelled selectively binding material are not particularly limited in terms of their kinds and production methods. For example, enzymes such as peroxidase or alkali phosphatase (hereinafter, which may be referred to as ALP) may be used as the labelling material. In this case, enzyme activity can be measured by using a substrate specific to the enzyme (for example, 1,2-phenylenediamine (hereinafter, which may be referred to as OPD) or 3,3',5,5'-tetramethyl benzidine in case of a horseradish peroxidase (hereinafter, which may be referred to as HRP), or p-nitrophenyl phosphate in case of ALP). In case where biotin is used as the labelling material, it is generally arranged such that biotin is reacted with avidin or an enzyme-modified avidin.

The sample used in the detection using the selectively binding material of the present invention is not particularly limited and various types of samples can be used. In exemplary aspects, biological samples such as liquid biological samples such as blood, blood plasma, blood serum, urine, and saliva, and solid biological samples such as skins, feces, and biopsy tissue can be used.

The selectively binding material of the present invention may be provided as a measuring reagent for use in the method of detecting a glycoprotein. The reagent may further include, apart from the selectively binding material of the present invention, the other constituent(s) necessary for carrying out the method of detecting, such as a buffer solution, or a preservative.

In one aspect, for example, in case where the selectively binding material of the present invention includes an enzyme as the labelling material, the selectively binding material of the present invention may be provided in the form of a kit together with a reagent including the substrate specific to the enzyme.

In case where a kit for use in the method of detecting a glycoprotein by using the selectively binding material of the present invention, a reagent for performing the enzyme treatment with the glycan-cleaving enzyme and a reagent for use in the step of obtaining the reaction product from the reaction between the glycoprotein and the selectively binding material may be provided as separate compositions, or the reagent for performing the enzyme treatment with the glycan-cleaving enzyme and the reagent for use in the step of obtaining the reaction product from the reaction between the glycoprotein and the selectively binding material may be provided as one composition. In one aspect, it is preferable that the reagent for performing the enzyme treatment with the glycan-cleaving enzyme and the reagent for use in the step of obtaining the reaction product from the reaction between the glycoprotein and the selectively binding material be provided as one composition.

### Examples

In the followings, the present invention will be described in more details referring to Examples, but the present invention is not limited to these Examples.

### Example 1

### 1. Treatment of Glycoprotein AFP-L3 with Endo-F3

Glycoprotein AFP affinity purified from culture supernatant of liver cancer cell strain HepG2 was treated with Endo-F3 (38.8 kDa, produced by New England Biolabs) under the following condition. The purified AFP includes AFP-L3.

### [Materials and Conditions]

(1) AFP: per 8 µg,
(2) Endo-F3: 16U (0.1 M MES buffer solution (pH 5.5) 20 µL was mixed and enzyme treated with

Treatment (enzyme reaction) Time Period: 10 min or 30 min

Treatment (enzyme reaction) Temperature: 37°C, to obtain enzyme-treated AFP liquid.

The said enzyme-treated AFP solution and Tris SDS sample treatment solution (manufactured by Cosmo Bio Co., Ltd., 423420) were added in equal amounts and boiled for 10 minutes. The boiled sample was electrophoresed at 30 mA for 70 minutes with Multigel (registered trademark) II Mini 4/20 (13 W) (manufactured by Cosmo Bio Co., Ltd., 414879), and then the gel was stained by conventional CBB staining.

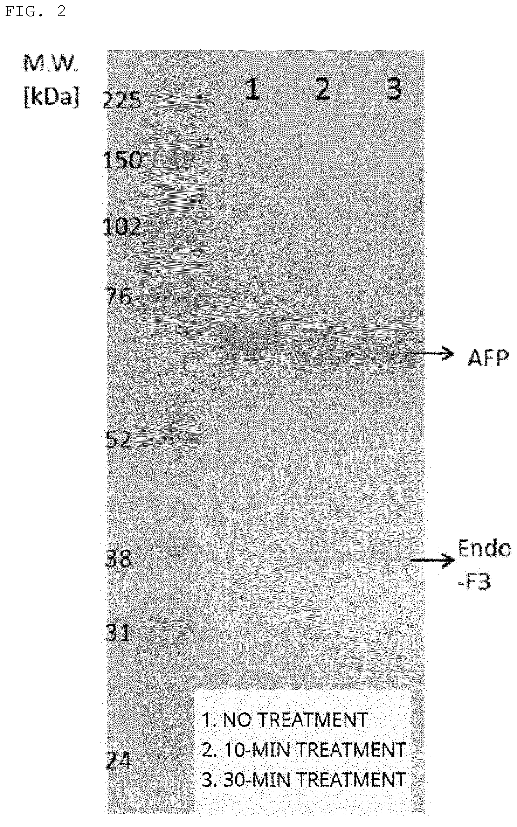

Fig. 2 illustrates results of electrophoresis (SDS-PAGE) of samples of no treatment, the treatment time period of 10 min, and the treatment time period of 30 min. The band detected between 52 kDa and 76 kDa in the non-treatment lane is a band derived from AFP, and the bands detected in the vicinity of 38 kDa in lanes 1 and 2 are bands derived from Endo-F3. Regarding the band derived from AFP, it was confirmed that the detected positions of bands of the treatment time periods 10 min and 30 min were shifted to lower molecular weights compared with the detected position of the band of no treatment. These results suggest that the treatment cleaved a fucose-containing glycan bound with AFP-L3.

### Example. 2

### 2. Preparation of Monoclonal Antibody

The AFP having the cleaved glycan as an antigen (immunogen) is inoculated to a mouse or rat by a known method, and a hybridoma was prepared from B cells obtained from a spleen of the mouse or rat.

The hybridoma is screened with the AFP having the cleaved glycan, thereby obtaining a monoclonal antibody that specifically detects the AFP having the cleaved glycan.

For more details, the experiment was carried out as below.

### [Materials]

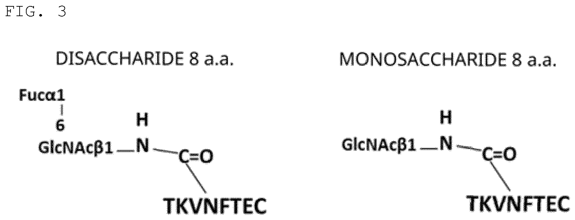

(1) Freund's Complete Adjuvant: made by Wako Pure Chemical Industries, Ltd., 014-09541
(2) Myeloma cells (SP2/O)
(3) RPMI1640, GlutaMAX: made by GIBCO, 61870-036
(4) Fetal Bovine Serum (FBS): made by BIOLOGICAL INDUSTRIES, 04-001-1A
(5) HAT medium: made by Cosmo Bio Co., Ltd. 16213004
(6) 96-well plate: NUNC, 167008
(7) HRP-labelled goat anti-mouse IgG (H&L) and HRP-labelled goat anti-rat IgG (H&L) antibody: made by Southern Biotech, 1031-05 and 3050-05
(8) Multigel (registered trademark) II mini 4/20 (13W): made by Cosmo Bio Co., Ltd., 414879
(9) Tris SDS sample treatment solution: made by Cosmo Bio Co., Ltd., 423420
(10) Glycopeptide: synthesized disaccharide 8a.a. and monosaccharide 8 a.a. (the sequences are shown in Fig. 3)

### [Experiment Example 1] Confirmation of Enzyme treatment

### 1. Experiment Method

Into 2 µL (5 to 7 µg) of AFP purified by affinity purification from a culture supernatant of HepG2, 2 µL (16U) of Endo-F2 (39.8 kD, made by New England Biolabs) or 2 µL (2 µg) of Endo-F3 was added, and 16 µL of 0.1 M MES buffer solution (pH 5.5) was further added. After these samples thus prepared were enzyme reacted at 37°C for 10 min, the same amount of tris SDS sample treatment solution was added therein and the samples were boiled for 10 min. These samples were added to Multigel (registered trademark) II mini 4/20 (13W) by 10 µL per lane, and electrophoresis was carried out at 30 mA for 70 min. After that, the gel was stained with usual CBB staining.

### 2. Experiment Result

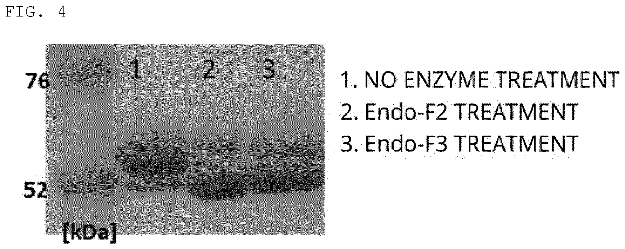

The results are shown in Fig. 4. Compared with the AFP without the glycan-cleaving enzyme, the AFPs with Endo-F2 or Endo-F3, which are glycan-cleaving enzymes, added therein showed different band patterns detected, and AFP treated with the enzyme had thicker band concentrations especially on the lower molecular weight side (Fig. 4). This indicates that some of glycans modified with AFP were digested and cleaved by the glycan-cleaving enzyme, thereby reducing the molecular weight. This result indicates that the glycan of AFP can be digested regardless of the types of the glycan-cleaving enzymes.

### [Experiment Example 2] Method of producing Monoclonal Antibody-1

### 1. Preparation of Immunogen

Disaccharide peptide imitating the amino acid and sugar chain sequences produced by glycan-cleaving enzyme treatment of AFP was cross-linked with various proteins such as KLH (Thermo, 77600) by using a commercially-available linker reagent, thereby preparing an immunogen solution.

### 2. Preparation of monoclonal antibody-producing hybridoma against glycan-cleaving enzyme-treated AFP

### (1) Inoculation to Animals

An emulsion prepared by mixing the same volumes of the immunogen solution (0.2 to 2 mg/mL) and Freund's complete adjuvant was injected as an antigen to Balb/cAJcl mice or F344 rats by 10 to 40 µg per animal. Further, the injection of the said emulsion was repeated 3 to 8 times with one-week interval. An antibody titer in an antiserum obtained by collecting blood from a tail vein was measured by antigen-immobilized ELISA method described later.

### (2) First Screening (Antigen-immobilized ELISA Method)

The presence of the antibody for the glycan peptide in the antiserum of the immunized animal was confirmed by ELISA method with an immobilized disaccharide peptide conjugate prepared in the same method as the immunogen (antigen-immobilized ELISA method). Details of the antigen-immobilized ELISA method are as below.

### (2-1) Preparation of Plate for Antigen-Immobilized ELISA Method

A conjugate solution containing the disaccharide peptide and a protein different from the one used for the preparation of immunogen was dissolved in a 20 mM phosphate buffer solution including 150 mM sodium chloride (pH 7.2; hereinafter, which is referred to as PBS) to attain a concentration of 1 µg/mL, and 50 µL of the solution thus prepared was inoculated in each well of 96-well microplate, and left stand at room temperature for 2 hours.

After each well was washed three times with 400 µL of PBS containing 0.05% Tween (registered trademark) 20 (hereinafter, which is referred to as PBST), 100 µL of PBST containing 1% bovine serum albumin (hereinafter, which is referred to as BSA-PBST) was added therein, and blocking was carried out at room temperature for 1 hour. The plate thus prepared was used as a plate for ELISA.

### (2-2) Antigen-immobilized ELISA Method

After each well of the plate for ELISA was washed three times with 400 µL of PBST, the antiserum of the immunized animal and serum of non-immunized animal, which were gradualdiluted with BSA-PBST were dispensed in each well of the ELISA plate by 50 µL, and left stand at room temperature for 1 hour. After each well was washed three times with 400 µL of PBST, a 10000-fold dilution of HRP-labelled anti-mouse IgG (H&L) diluted with BSA-PBST or a 5000-fold dilution of HRP-labelled rat IgG (H&L) diluted with BSA-PBST was inoculated in each well by 50 µL, and left stand at room temperature for 1 hour. After each well was washed three times with 400 µL of PBST, 50 µL of citrate buffer solution (pH 5.0) containing 0.2% o-phenylenediamine and 0.02% hydrogen peroxide was added therein and let stand at room temperature for 10 min. After that, 50 µL of 1.5 N sulfuric acid was added therein to stop enzyme reaction, and absorbance at wavelength of 492 nm was measured. From a mouse or rat found to have a high antibody titer as a result of the measurement, a spleen and lymph node were removed to prepare spleen-derived or lymph node-derived cells, and the cells were used for cell fusion.

### (2-3) Experiment Result

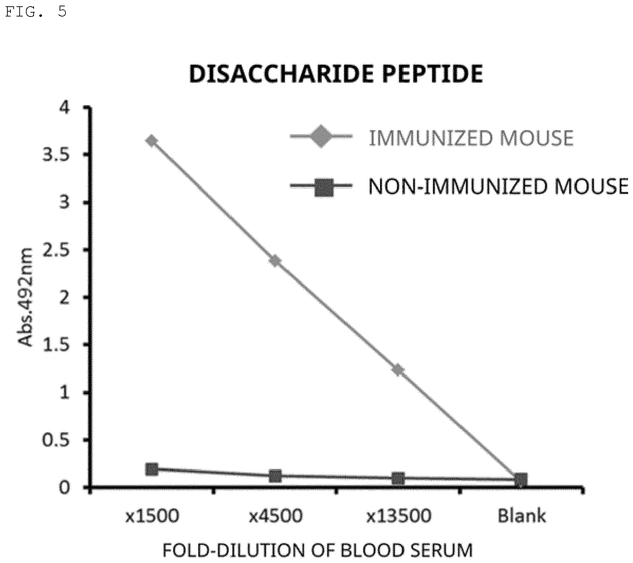

The result from immunization of mouse is shown in Fig. 5 as an example of the results. Compared with the serum of the non-immunized animal, the anti-serum of immunized animal showed the higher absorbance, the lower the dilution rate, only when the disaccharide glycopeptide was immobilized and used. In this measurement system, the absorbance measured depends on the concentration of the antibody against the disaccharide peptide contained in the serum of the animal from which the blood was collected (Fig. 5). Accordingly, this result demonstrates that the antiserum of the immunized animal showed greater antibody titer since the serum contained a greater concentration of the antibody against the disaccharide peptide compared with the serum of the non-immunized animal.

### (3) Cell Fusion

Either the spleen-derived cells or the lymph node-derived cells were mixed with myeloma cells at a cell number ratio of 1 : 1, and fused together by electric pulse method. The cells thus fused were dispersed in a HAT medium and incubated at 37°C in 5% CO₂ for 8 days in a CO₂ incubator, thereby obtaining fused cells (hybridoma).

### (4) Selection of Hybridoma (Antigen-immobilized ELISA Method)

### (4-1) Method

A similar antigen-immobilized ELISA method was carried out except that, instead of using the serum of the immunized animal, a culture supernatant of the fused cells was used, and that each conjugate of monosaccharide peptide, enzyme untreated AFP and Endo-F3 treated AFP were used as immobilized antigens in addition to the conjugate of disaccharide peptide. As a result of the measurement, wells showed a higher absorbance for the disaccharide peptide than the monosaccharide peptide or wells showed a higher absorbance for the AFP treated with Endo-F3 than the AFP not treated with enzyme was selected as a well in which the anti-disaccharide peptide or the anti-enzyme treatment AFP antibody-producing hybridoma was present (positive well).

### (4-2) Experiment Result

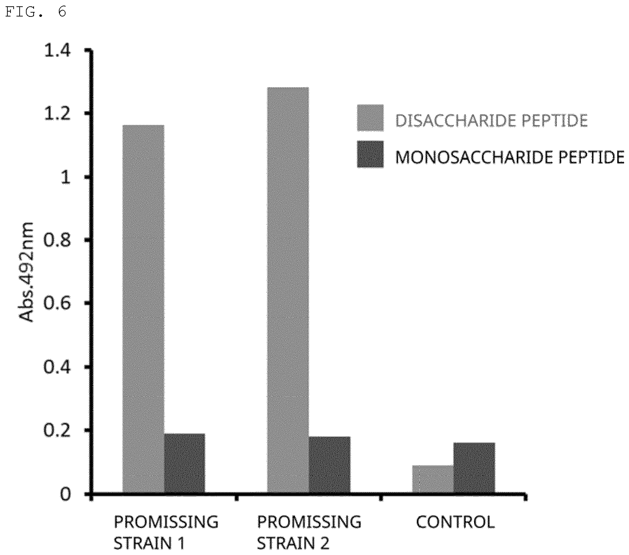

The result from the experiment where disaccharide peptide or monosaccharide peptide was immobilized are shown in Fig. 6 as an example of the results. In the animal inoculated with the disaccharide peptide, a plurality of antibody-producing strains reactive with the disaccharide peptide but not with monosaccharide peptide was confirmed (Fig. 6). This result demonstrates that the use of disaccharide peptide as the immunogen makes it possible to obtain the antibody-producing hybridoma for a region in the vicinity of the disaccharide-glycan-modified site of the enzyme-treated AFP.

### [Experiment Example 3] Method of producing Monoclonal Antibody-2

### 1. Experiment Method

The AFP purified from the culture supernatant of HepG2 was treated with Endo-F3 as in Experiment Example 1, and treated as in Experiment Example 2, using, as an immunogen, a sample from which the enzyme was removed. However, the antigen-immobilized ELISA method was carried out by using, as the antigen, AFP treated with Endo-F3.

### 2. Experiment Result

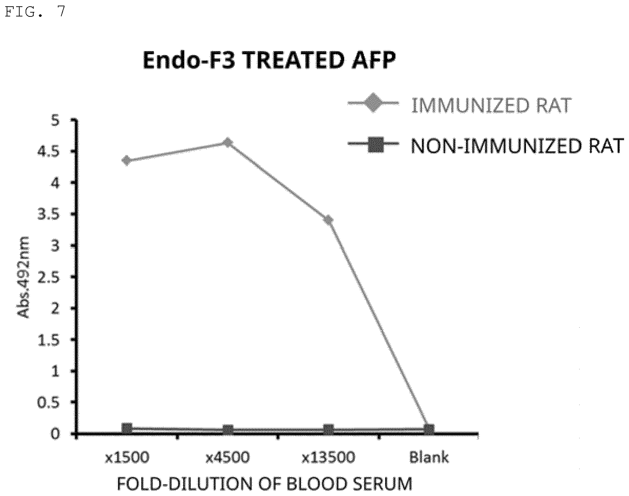

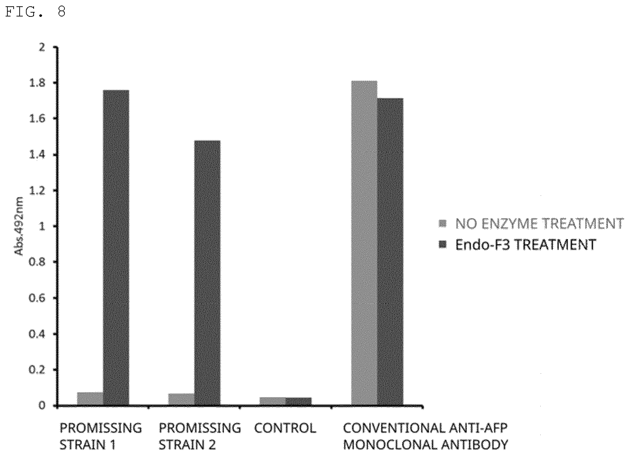

The result from the experiment where rat was inoculated is shown in figures 7 and 8 as an example of the results. Compared with the serum of the non-immunized animal, the antiserum of the immunized animal showed the higher absorbance for the immobilized enzyme-treated AFP, the lower the dilution rate of the antiserum (Fig. 7). In this measurement system, the absorbance measured depends on the antibody concentration against the enzyme-treated AFP contained in the serum of the animal from which the blood was collected. That is, this result demonstrates that the antiserum of the immunized animal contained a greater concentration of the antibody against the enzyme-treated AFP compared with the non-immunized animal serum, thereby having a greater antibody titer.

As illustrated in Fig. 8, in the animal inoculated with the enzyme-treated AFP, a plurality of antibody-producing strain reactive with the enzyme-treated AFP but not reactive with the AFP not treated with the enzyme treatment. That is, this demonstrated that the use of the enzyme-treated AFP as the immunogen makes it possible to obtain an antibody-producing hybridoma specific to the enzyme-treated AFP.

### [Experiment Example 4] Cloning

Using the antibody-producing strain hybridoma thus selected via the first screening and the second screening, monocloning of the hybridoma and monoclonal antibody purification were carried out. The cloning was performed according to a usual method (Limiting Dilution Method, see, for example, *"*Jikken Igaku Bessatsu, Tanpakushitsu Jikken Handbook" ("Experimental Medicine Separate Volume, Protein Experiment Handbook") ISBN4-89706-369-8), positive wells were selected by the same method as the antigen-immobilized ELISA method, thereby finally obtaining two types of monoclonal antibody-producing hybridomas (S20205R and S20206R).

### [Experiment Example 5] Specificity Analysis of Monoclonal Antibody of the Present Invention 1

How specifically the antibodies that S20205R and S20206R produce reacted with the enzyme-treated AFP was confirmed by immuno-precipitation and Western blotting.

### 1. Experiment Method

Into 400 µL of a culture supernatant of the HepG2 cells, 400 µL of a 0.1M MES buffer solution and further 7.5 µg of Endo-F3 were added, a mixture thus prepared was reacted at 37°C for 30 min. After that, 800 µL of BSA-PBST was added therein to prepare the test sample. Then, 400 µL of the sample thus prepared was added in 100 µL of each of Sepharose gels (GE healthcare: CNBr activated Sepharose 4 Fast Flow (17098101)) respectively with 4 types of antibodies, namely, S20205R antibody, S20206R antibody, a conventional anti-AFP antibody (manufactured by the applicant), and an antibody not reactive with AFP immobilized thereon by a usual method, and stirred at room temperature for 2 hours. After 10000 g of them were centrifuged for 2 min to remove a supernatant therefrom, and the remaining is dispersed in 1 mL of PBST, and 10000 g thereof was centrifuged again for 2 min. After this operation was repeated twice and a supernatant was removed therefrom, 60 µL of tris SDS sample treatment solution was added therein and a mixture thus prepared was boiled for 10 min. After that, 10000 g thereof was centrifuged for 2 min, and SDS-PAGE was carried out in a similar manner as in [Experiment Example 1], adding 15 µL per lane. The operation up to this point is referred to as immunoprecipitation. The gel after the said electrophoresis was transferred to a PVDF membrane by a usual method. In 1% BSA-PBST, the PVDF membrane was vibrated at room temperature for 1 hour. After that, in 1% BSA-PBST in which the conventional anti-AFP antibody or the antibody not reactive with AFP labelled with biotin as the detection antibody was diluted to 1 µg/mL, the PVDF membrane was vibrated at room temperature for 1 hour. Next, the PVDF membrane was vibrated at room temperature for 5 min in PBST, and a supernatant was removed therefrom. After this operation was repeated three times, the PVDF membrane was vibrated at room temperature for 30 min in 1% BSA-PBST in which HRP-labelled Streptavidin was diluted to 0.33 µg/mL. Next, the PVDF membrane was vibrated at room temperature for 5 min in PBST, and a supernatant was removed therefrom. After this operation was repeated three times, the PVDF membrane was immersed in 50 mM Tris hydrochloride buffer solution (pH 7.4) containing diaminobenzidine of 0.2 mg/mL and 0.0012% of hydrogen peroxide. After a band was visually confirmed sufficiently, the PVDF membrane was washed with RO water.

### 2. Experiment Result

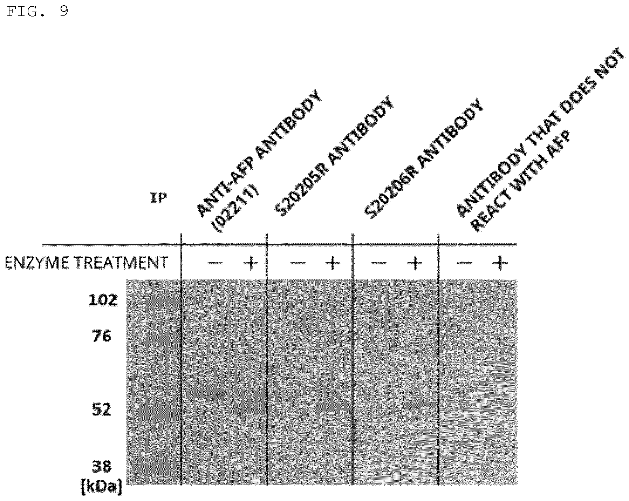

The result is shown in Fig. 9. In case where the immunoprecipitation was performed with the conventional anti AFP antibody and the detection was performed with the anti-AFP antibody (left lane), a band was detected regardless of whether the enzyme treatment was carried out (- and + lanes of the left lane). Further, it was confirmed that, in case where the enzyme treatment was carried out, two bands were observed respectively on the higher molecular weight side and the lower molecular weight side (+ lane of the left lane). On the other hand, in case where the immunoprecipitation was carried out with S20205R antibody (center left lane) or S20206R antibody (center right lane) and the detection was carried out with the anti-AFP antibody, a band was only detected on the lower molecular weight side if the enzyme treatment was carried out (each + lane of center left and center right lanes) (Fig. 9). In this measurement system, the band of AFP to which the antibody used in the immunoprecipitation binds is detected. That is, as demonstrated in [Experiment Example 1], this demonstrates that the enzyme treatment expanded the band on the lower molecular weight side, and S20205R antibody or S20206R antibody recognizes the glycolysis AFP caused by the enzyme treatment. Note that no substantial bands were detected for the antibody that does not react with AFP (right lane).

### [Experiment Example 6] Specificity Analysis of Monoclonal Antibody of the Present Invention 2

### 1. Experiment Method

The specificities of S20205R antibody and S20206R antibody were confirmed by the same method as the antigen-immobilized ELISA method. Note that, the antigens immobilized on the plates were AFP, AFP treated with Endo-F3, and AFP treated with enzyme PNGaseF (an enzyme that cleaves the bond between the amino group and GlcNAcβ1 of the sequence shown in figure 3). At the same time, SDS-PAGE of each AFP was conducted as in [Experiment example 1]

### 2. Experiment Result

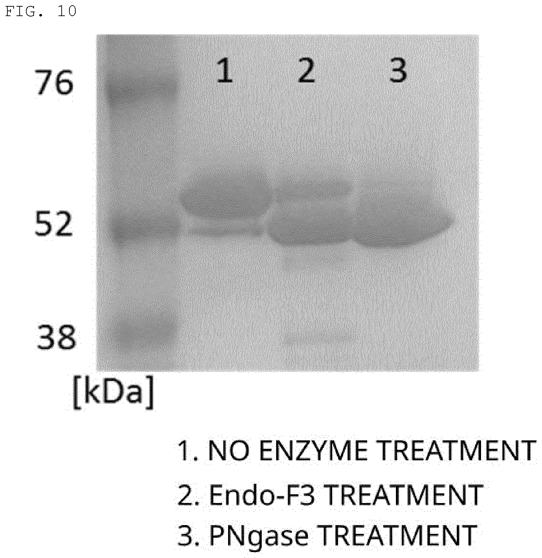

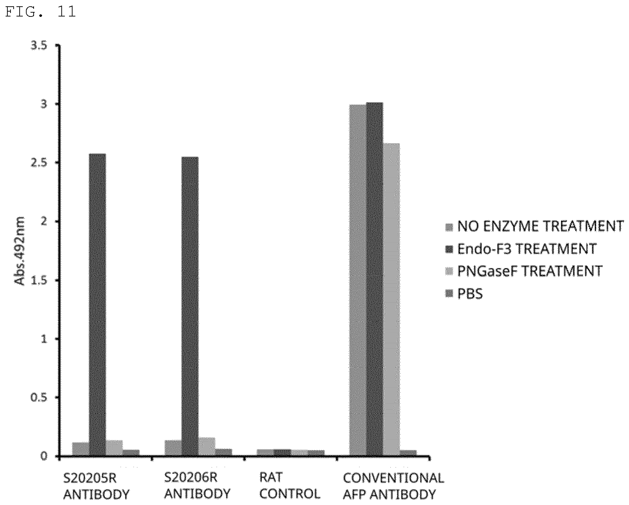

Results are shown in Figs. 10 and 11. In SDS-PAGE, the AFP without the enzyme treatment, the AFP treated with Endo-F3, and the AFP with PNGaseF treatment showed different band patterns. The AFP treated with Endo-F3 showed two bands respectively on the higher molecular weight side and the lower molecular weight side, while a band was confirmed on the lower molecular weight side for the AFP treated with PNGaseF (Fig. 10). This demonstrated that Endo-F3 cleaved some of the glycan of the AFP leaving disaccharides, whereas PNGaseF digested and cleaved all N-type glycans, producing an AFP without glycan. On the other hand, as illustrated in Fig. 11, while the conventional AFP antibody showed an increase in absorbance regardless of whether or not the enzyme treatment was carried out, S20205R antibody and S20206R antibody showed such an increase in absorbance only for the AFP treated with Endo-F3. In this measurement system, the greater reactivity of the antibody against the AFP immobilized on the plates is, the greater the absorbance becomes. That is, this demonstrates that S20205R antibody and S20206R antibody include the glycan caused by Endo-F3 as a part of the recognition site.

### [Experiment Example 7] Sandwich ELISA with Conventional Antibody

So far, the reactivity against the antigens immobilized on the plates had been evaluated, but reactivities against antigens present in a liquid phase were also evaluated. An experiment method was as below.

### 1. Experiment Method

### (1) Preparation of Plates for Sandwich ELISA

A conventional anti-AFP monoclonal antibody (made by Sekisui Medical Co., Ltd.) being reactive regardless of whether or not the enzyme treatment was carried out was prepared by using purified AFP as an immunogen and a solution of conventional anti-AFP monoclonal antibody was dissolved in a 20 mM phosphate buffer solution containing 150 mM sodium chloride (pH 7.2; hereinafter, which is referred to as PBS) in such a manner that concentration of the anti-AFP monoclonal antibody became 5 µg/mL. The solution thus prepared was dispensed in each well of a 96-well microplate by 50 µL, and left stand at room temperature for 2 hours.

After each well was washed three times with 400 µL of PBS containing 0.05% Tween (registered trademark) 20 (hereinafter, which is referred to as PBST), 100 µL of PBST containing 1% bovine serum albumin (hereinafter, which is referred to as BSA-PBST) was added therein, and blocking was carried out at room temperature for 1 hour. The plate thus prepared was used as a plate for sandwich ELISA.

### (2) Sandwich ELISA Method

As in [Experiment Example 1], gradual dilutions of AFP treated with Endo-F3 were prepared with BSA-PBST. Those are referred to as antigen-containing solution hereinafter. After the solution was removed from each well of the sandwich ELISA plate, the antigen-containing solution was dispensed in each well by 50 µL, and left stand at room temperature for 15 min. After each well was washed three times with 400 µL of PBST, a 1500-fold dilution of HRP-labelled S20205R antibody or HRP-labelled S20206R antibody diluted with BSA-PBST was dispensed in each well of the plate by 50 µL, and left stand at room temperature for 15 min. After each well was washed three times with 400 µL of PBST, 50 µL of citrate buffer solution (pH 5.0) containing 0.2% o-phenylenediamine and 0.02% hydrogen peroxide was added therein and let stand at room temperature for 10 min. After that, 50 µL of 1.5 N sulfuric acid was added therein to stop enzyme reaction, and absorbance at wavelength of 492 nm was measured.

### 2. Experiment Result

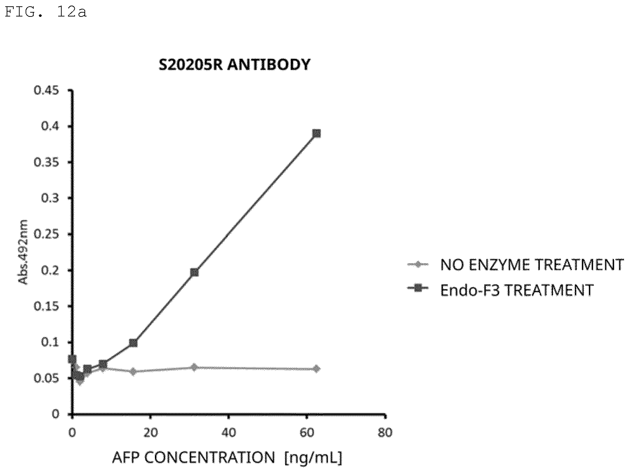

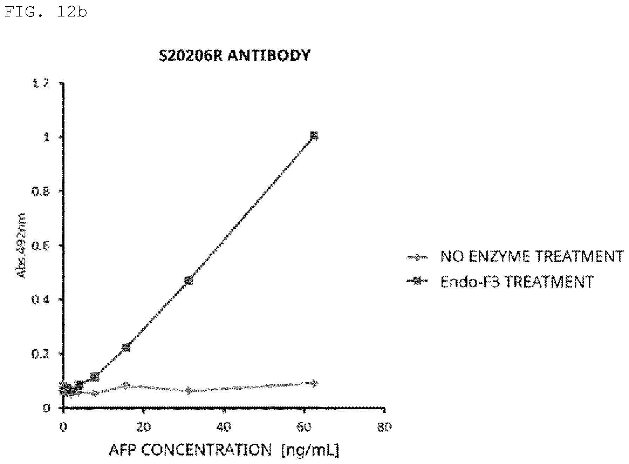

Results are shown in Figs. 12a and 12b. The absorbance increased dependently on the concentration of the enzyme-treated AFP (Figs 12a and 12b). In this measurement system, the absorbance is increased as a result of reaction of the HRP-labelled S20205R antibody and HRP-labelled S20206R antibody with the enzyme-treated AFP being in the liquid phase and bound with the conventional anti-AFP antibody immobilized on the plate. That is, this demonstrates that these antibodies specifically react with the enzyme-treated AFP in the liquid phase.

The results so far are such that S20205R antibody and S20206R antibody were substantially identical in terms of characteristics, therefore, evaluations below used only S20206R antibody.

### [Experiment Example 8] Reactivity Analysis with respect to each Enzyme Treatment Product by Sandwich ELISA

### 1. Experiment Method

The experiment method herein is similar to [Experiment Example 7]. However, the antigen was AFP treated with Endo-F2 or Endo-F3 prepared as in [Experiment Example 1]. Moreover, In the process of adding the HRP-labelled antibody in [Experiment Example 7], a 6000-fold dilution of HRP-labelled conventional anti-AFP polyclonal antibody diluted with BSA-PBST was also used in the experiment.

### 2. Experiment Result

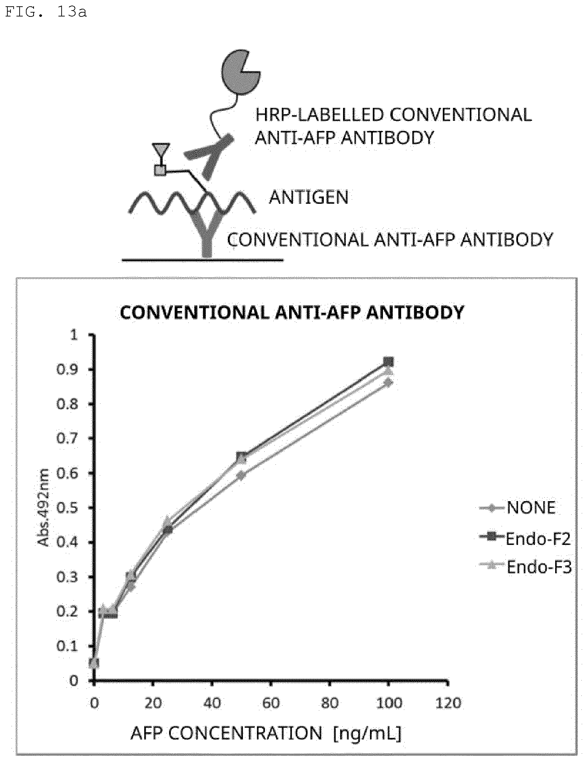

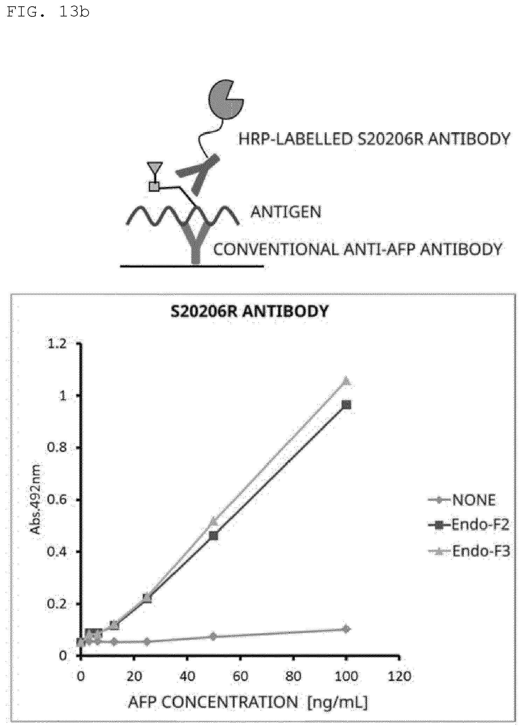

Results are shown in Figs. 13a and 13b. In case where the measurement was carried out with the conventional AFP antibody against another conventional AFP antibody, the absorbance increased dependently on the AFP concentration regardless of whether or not the enzyme treatment was carried out (Fig. 13a). On the other hand, in case where S20206R antibody was used, the absorbance increased dependently on the concentration of only the AFP treated with Endo-F2 or Endo-F3 (Fig. 13b). That is, this demonstrates that the use of Endo-F2 also produced a similar glycolysis AFP to that produced by the use of Endo-F3, and that the use of an enzyme other than Endo-F3 also makes it possible to perform similar measurement as in the Experiment Examples above.

### [Experiment Example 9] Study on Enzyme Treatment in Blood Serum

So far, the enzyme treatment had been performed by adding the enzyme and the buffer solution in the AFP purified. However, detection samples actually assumed in the clinical test is blood serum and it is expected that blood serum contains a very high concentration of N-type glycan protein besides AFP. Thus, it was tested whether or not the enzyme acts on AFP even in blood serum.

### 1. Experiment Method

The experiment method herein is similar to [Experiment Example 7]. However, the antigen used prepared by adding 25 µL of blood serums of three health persons in 200 ng of AFP derived from HepG2, and further 25 µL of 0.1 M MES buffer solution (pH 5.5) and 2 µg of Endo-F3 were added therein. After a mixture thus prepared was reacted at 37°C for 10 min, 1 mLmL of BSA-PBST was added therein to adjust AFP concentration to 200 ng/mL, thereby preparing an antigen-containing solution.

### 2. Experiment Result

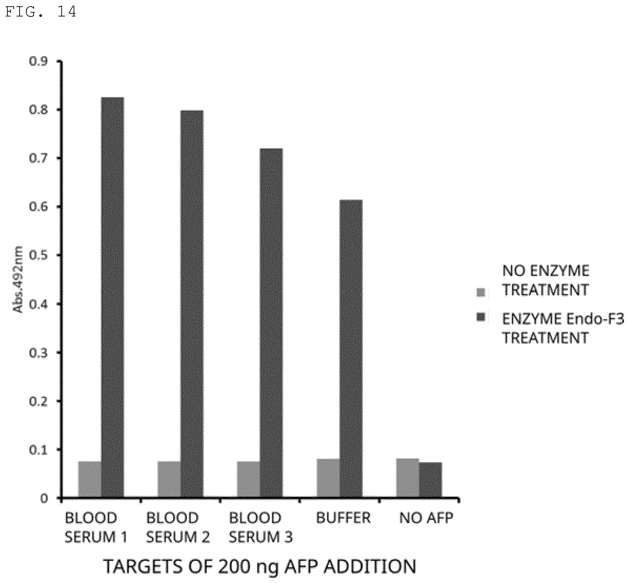

The result is shown in Fig. 14. In case AFP was added in the blood serum of healthy persons, as in the case of adding AFP in the buffer solution, the sandwich ELISA according to the present invention showed an increase of absorbance specifically to the enzyme treatment AFP (Fig. 14). That is, this demonstrates that Endo-F3 also affect AFP also in blood serum.

### [Experiment Example 10] Study on pH condition of Enzyme Treatment

### 1. Experiment Method

So far, 0.1 M MES buffer solution (pH 5.5) had been used as the buffer solution in most of the cases, but it was studied whether or not other buffer solutions (pH) were also usable. The experiment method herein is similar to [Experiment Example 7]. However, the buffer solutions listed on Table 1 were used in the enzyme treatment.

**[Table 1]**

| Buffer used | pH |
|---|---|
| 1 N HCl | 0.3 |
| 0.1 M Gly-HCl | 2 |
| | 2.5 |
| | 3 |
| | 3.5 |
| 40mM Sodium acetate | 4 |
| | 4.5 |
| | 5 |
| 0.1 M MES | 5.5 |
| | 6 |
| | 6.5 |
| 20 mM Tris | 7 |
| | 7.5 |
| | 8 |
| 0.1 M CHES | 8.5 |
| | 9 |
| | 9.5 |
| 0.1 M CAPS | 10 |
| | 10.5 |
| | 11 |
| 1N NaOH | 13.3 |

After the enzyme treatment, the solution was diluted with BSA-PBST to adjust the AFP concentration to 100 ng/mL, and used for the sandwich ELISA.

### 2. Experiment Result

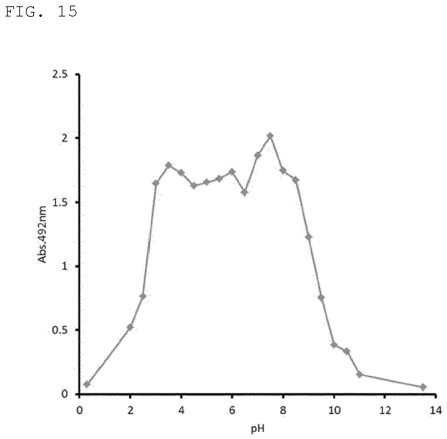

Results are shown in Fig. 15. The absorbance was 0.1 or more in the region from about pH 0.5 to about pH 12.5, indicating that reaction took place (Fig. 15). This sufficiently satisfied the pH region of normal buffer solutions used in molecular biology, and therefore, the results demonstrate that the enzyme of the present invention can be used at any pH.

### [Experiment Example 11] Study on on-plate enzyme treatment.

So far, the measurement was carried out with sandwich ELISA after diluting AFP with BSA-PBST after the enzyme treatment. Here, whether or not the enzyme treatment of AFP could be performed at the same time with the antigen-antibody reaction in the sandwich ELISA was tested.

### 1. Experiment Method

The experiment method was similar to [Experiment Example 7] until the plate preparation.

### (1) On-plate Enzyme Treatment.

Endo-F3 was diluted with 0.1 M MES buffer solution (pH 5.5) to 0.33 mg/mL, thereby preparing an enzyme-containing solution. Moreover, gradual dilutions of a culture supernatant of HepG2 containing AFP diluted with 0.1 M MES buffer solution (pH 5.5) were prepared, thereby preparing antigen-containing solutions.

After the liquid was removed from each well of the plate for ELISA, 30 µL of the enzyme-containing solution was added in each well. Next, 30 µL of the antigen-containing solutions was added in each well, and reacted at 37°C for 20 min. After each well was washed three times with 400 µL of PBST, a 1500-fold dilution of HRP-labelled S20206R antibody diluted with BSA-PBST was dispensed in each well of the plate by 50 µL, and left stand at room temperature for 15 min. After each well was washed three times with 400 µL of PBST, 50 µL of citrate buffer solution (pH 5.0) containing 0.2% o-phenylenediamine and 0.02% hydrogen peroxide was added therein and let stand at room temperature for 10 min. After that, 50 µL of 1.5 N sulfuric acid was added therein to stop enzyme reaction, and absorbance at wavelength of 492 nm was measured.

### 2. Experiment Result

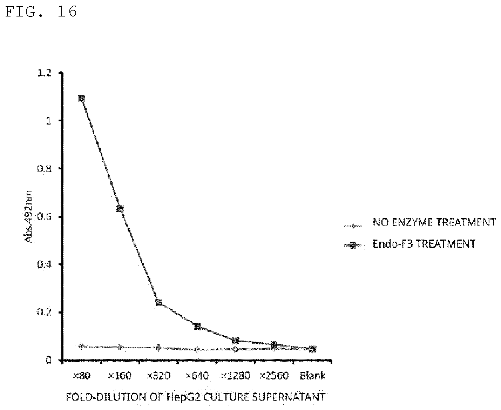

Results are shown in Fig. 16. As in the results so far, the addition of the enzyme resulted in an increase of the absorbance in a concentration-dependent manner (Fig. 16). In this measurement system, the antigen and the enzyme react with each other concurrently with the reaction between the immobilized antibody and the antigen. That is, this demonstrates that the antigen-antibody reaction and the enzyme treatment reaction of the antigen can be performed at the same time.

Even though the present invention has been described referring to the concreate Examples, but the scope of the present invention is not limited to these concrete Examples. A person skilled in the art will understand that various modifications can be adopted within the gist of the present invention.

## Claims

1. A method of treating a glycoprotein to when the glycoprotein is used as an antigen,
the method comprising:
a step of cleaving a glycan with a glycan-specific glycan-cleaving enzyme.

2. The method of treating a glycoprotein according to claim 1,
wherein the step of cleaving the glycan
causes an epitope present in the glycan to be recognizable with a selectively binding material.

3. The method of treating a glycoprotein according to claim 1 or 2, wherein the glycan-specific glycan-cleaving enzyme is one selected from the group consisting of Endo-A, Endo-M, Endo-H, Endo-D, Endo-F1, Endo-F2, and Endo-F3.

4. The method of treating an antigen according to any one of claims 1 to 3, wherein the glycoprotein is AFP.

5. A method of producing a selectively binding material, wherein the method of treating a glycoprotein according to any one of claims 1 to 4 is employed.

6. A method of detecting a glycoprotein in a biological sample, wherein the method of treating a glycoprotein according to any one of claims 1 to 4 is employed.

7. A glycoprotein treated by the method of treating a glycoprotein according to any one of claims 1 to 4.

8. A selectively binding material being bindable with a glycoprotein treated by the method of treating a glycoprotein according to any one of claims 1 to 4 and not bindable with the same glycoprotein not treated as such.

9. The selectively binding material according to claim 8, being an antibody or an antigen-binding fragment of an antibody.

10. A method of producing the selectively binding material according to claim 8 or 9, the method comprising:
employing, as an antigen, a glycoprotein treated with the method of treating an antigen according to any one of claims 1 to 4.

11. A method of producing the selectively binding material according to claim 8 or 9, the method comprising:
employing, as an antigen, a glycoprotein obtained by chemical synthesis or gene recombination technique.

12. A method of detecting a glycoprotein,
the method comprising:
a step of treating, with a glycan-cleaving enzyme, a sample that potentially contains the glycoprotein;
a step of applying the selectively binding material according to claim 8 to the glycoprotein treated with the glycan-cleaving enzyme, so as to obtain a reaction product of a reaction between the glycoprotein and the selectively binding material; and
a step of detecting the reaction product.

13. The method according to claim 12, wherein the step of treating the glycoprotein with the glycan-specific glycan-cleaving enzyme and the step of obtaining the reaction product of the reaction between the glycoprotein and the selectively binding material are carried out at the same time.

14. A reagent kit for use in the method according to claim 12 or 13, wherein a reagent for treating a glycoprotein with a glycan-specific glycan-cleaving enzyme and a reagent for obtaining a reaction product of the glycoprotein and a selectively binding material are included in a single composition.

15. A method of producing an antibody by using an antigen having a glycan structure not present in vivo or very rare in vivo even if the glycan structure is present in vivo.

16. The method according to claim 15, wherein the antigen is a glycoprotein treated with a glycan-specific glycan-cleaving enzyme.

17. The method according to claim 15, wherein the antigen is one obtained by chemical synthesis or gene recombination technique.
